# EUROPÄISCHE PATENTSCHRIFT

(11) **EP 1 040 168 B1**
(45) Veröffentlichungstag und Bekanntmachung des Hinweises auf die Patenterteilung: **06.11.2002**
(21) Anmeldenummer: 98965849.7
(22) Anmeldetag: 17.12.1998
(51) Int. Cl.: C09B 69/00, C08B 37/00, C09B 69/10, G01N 33/58, G01N 33/52

(54) **NEUE FARBSTOFF-POLYSACCHARID-KONJUGATE UND DEREN VERWENDUNG ALS DIAGNOSTIKUM**
NOVEL DYE-POLYSACCHARIDE-CONJUGATES AND THE UTILIZATION THEREOF AS A DIAGNOSTIC
NOUVEAUX CONJUGUES COLORANT-POLYSACCHARIDE ET LEUR UTILISATION COMME AGENT DIAGNOSTIQUE

(30) Priorität: 17.12.1997 EP 97122248
(43) Veröffentlichungstag der Anmeldung: 04.10.2000
(73) Patentinhaber: Roche Diagnostics GmbH, 68305 Mannheim (DE)
(72) Erfinder: BOSIES, Elmar, D-69469 Weinheim (DE); HEIN, Heinz-Michael, D-64342 Seeheim-Jugenheim (DE); REITER, Rudolf, D-69469 Weinheim (DE); JOSEL, Hans-Peter, D-82362 Weilheim (DE)
(86) Internationale Anmeldenummer: EP9808282
(87) Internationale Veröffentlichungsnummer: WO99031183

(56) Entgegenhaltungen:
- EP-A- 0 543 333
- DE-A- 4 436 164
- FR-A- 2 015 894
- FR-A- 2 757 162
- GB-A- 2 302 094
- US-A- 3 304 297
- US-A- 3 679 661
- US-A- 3 852 413
- US-A- 4 966 607
- CHEMICAL ABSTRACTS, vol. 118, no. 11, 15. März 1993 Columbus, Ohio, US; abstract no. 97152s, XP002064575 & CS 274 948 A (SLOVENSKA TECHNICKA UNIVERSITA)
- R. GUETHER AND M.V. REDDINGTON: "photostable cyanine dye beta-cyclodextrin conjugates" TETRAHEDRON LETTERS, Bd. 38, Nr. 35, - 1997 Seiten 6167-6170, XP002064574
- CHEMICAL ABSTRACTS, vol. 85, no. 13, 27. September 1976 Columbus, Ohio, US; abstract no. 87998h, XP002064576 & N. PAREKH ET AL: "renal test dyes" PFLUEGERS ARCH., Bd. 364, Nr. 1, - 1976 Seiten 77-81,

## Beschreibung

Die vorliegende Erfindung betrifft neue Farbstoff-Polysaccharid-Konjugate sowie deren Verwendung als Diagnostikum, insbesondere zur Bestimmung der glomerulären Filtrationsrate beim Menschen.

Im Zusammenhang mit diagnostischen und therapeutischen Maßnahmen im präklinischen und klinischen Bereich kommt der Bestimmung der glomerulären Filtrationsrate eine große Bedeutung zu, weil sich mit ihrer Hilfe die Einschränkung der Nierenfunktion bestimmen läßt. Unter der glomerulären Filtrationsrate (GFR) versteht man die von den Glomerula der Nieren pro Zeiteinheit produzierte Menge Primärharn, die mit jedem Stoff ermittelt werden kann, der ausschließlich filtriert wird und weder tubulär sezerniert noch aus dem Primärharn rückresorbiert wird.

Zur Quantifizierung der glomerulären Filtrationsrate wird die Clearance der verwendeten Testsubstanz untersucht. Als Clearance bezeichnet man diejenige Plasmamenge in ml, die durch die Nieren in der Zeiteinheit (min) von einer bestimmten (Test-) Substanz befreit wird.

Es werden zur Zeit verschiedene Methoden angewendet, um diese Clearance zu bestimmen. So haben die endogene Kreatinin-Clearance, die Inulin-Clearance oder die ⁵¹Cr-EDTA (Na₂ ⁵¹Cr-ethylendiamintetraacetat)-Clearance Bedeutung erlangt. Auch Röntgenkontrastmittel wie Iohexol werden zur Bestimmung der Nierenclearance eingesetzt.

Alle aufgeführten Methoden haben jedoch gravierende Nachteile. So ist je nach Ausführungsart der jeweiligen Clearance-Bestimmung eine Dauerinfusion der Testsubstanz zur Aufrechterhaltung eines konstanten Plasmaspiegels und/oder ein mit unangenehmer Katheterisierung verbundenes Sammeln von Urin und/oder eine mehrmalige Blutentnahme nötig. Die Bestimmung der Inulinkonzentration im Plasma oder Urin ist ein aufwendiges Verfahren und mit einer relativ großen Fehlerbreite behaftet. Die Verwendung von radioaktiv markierten Substanzen stellt eine zusätzliche Belastung für den Organismus dar und sollte daher möglichst vermieden werden.

Als eleganteste Form der Clearance-Bestimmung wird die sogenannte Input-Clearance angesehen. Hierbei wird nicht die Ausscheidung eines Markers direkt gemessen, sondern man hält die Plasmakonzentration des Markers konstant. Die erforderliche Menge an Marker, die benötigt wird, um die Plasmakonzentration konstant zu halten, entspricht der über die Nieren ausgeschiedenen Markermenge und damit der Clearance. Hierbei muß naturgemäß die Plasmakonzentration kontinuierlich überwacht werden, was heutzutage nur mit Hilfe radioaktiv markierter Substanzen machbar ist.

Eine deutliche Verbesserung läge in der Verwendung von nicht radioaktiv markierten Markern, deren Plasmakonzentration kontinuierlich überwacht werden könnte. Hierdurch könnte auch auf die Blutentnahme bzw. das Urinsammeln zur Konzentrationsbestimmung des Markers verzichtet werden.

Die Aufgabe bestand daher darin, einen nicht radioaktiv markierten Marker zu finden, der vollständig durch renale Ultrafiltration eliminiert wird, also weder tubulär sezerniert noch rückresorbiert wird, und dessen Plasmakonzentration durch ein invasives oder nicht invasives Verfahren kontinuierlich bestimmt werden kann, wobei das nicht invasive Verfahren bevorzugt ist und die Bestimmung der Plasmakonzentration insbesondere durch transkutane Absorptionsmessungen vorgenommen wird.

Die vorliegende Erfindung löst dieses Problem durch Verwendung von Polysacchariden, die kovalent mit einem Farbstoff verknüpft sind, wobei die Plasmakonzentration des Gesamtmoleküls mit Hilfe eines Detektors transkutan durch Absorptionsmessung bestimmt wird.

In der Literatur sind bereits einige derartige Verbindungen beschrieben. So gibt es Konjugate des Farbstoffes Cibacron Blau mit Amylose, Glucanen und Cellulose (Anal. Biochem. 31, 412 (1969); Acta Chem. Scand. 25, 298 (1971)); Biochem. J. 87, 90 (1963), das sogenannte Blue Dextran (Anal. Biochem. 39, 202 (1971) sowie den Farbstoff Remazole Brilliant Blue R in Verknüpfung mit Amylose (Experientia 23, 805 (1967)). In all diesen Farbstoff-Polysaccharid-Konjugaten handelt es sich bei dem Polysaccharid um ein aus Glucoseeinheiten aufgebautes Polysaccharid. Jedoch werden diese zum einen relativ schnell metabolisch abgebaut und eignen sich daher nicht für die Bestimmung der Nierenclearance. Zum anderen werden solche Polysaccharide nicht nur durch glomeruläre Filtration durch die Niere ausgeschieden und sind daher auch aus diesem Grunde für die Bestimmung der Nierenclearance nicht geeignet.

Als Polysaccharide im Sinne der vorliegenden Erfindung finden nun vor allem Polyfructosane wie z.B. Inulin, Levan, Asparagosin, Sinistrin, Fibrulin, Graminin, Phlein, Poan, Secalin und Irisin Verwendung, die durch glomuläre Filtration über die Niere ausgeschieden werden. Hierbei handelt es sich um Substanzen mit Kettenlängen von 10 - 30 Fructoseeinheiten, die teilweise am Ende einer Kette ein Glucosemolekül tragen. Vor allem werden Polysaccharide vom Typ des Inulins bzw. des besser wasserlöslichen Sinistrins eingesetzt.

Aus Chem. Abstracts Vol. 118, No. 11, Abstr. No. 97152a, sind Inulinkonjugate mit Anthrachinonen bekannt, die zur Aktivitätsbestimmung von Inulase einsetzbar sind. GB-A 2,302,094 offenbart unterschiedliche Reaktivfarbstoffe, die an unterschiedliche biologische Moleküle, u.a. Inulin, konjugiert sein können.

Bei der Farbstoffkomponente der Konjugate werden Farbstoffe eingesetzt, die eine funktionelle Gruppe wie z.B. eine Carboxyl-, Hydroxysulfonyl-, Amino-, Isothiocyanato- oder Isocyanatogruppierung tragen. Diese funktionellen Reste können direkt mit einem Polysaccharid zur Umsetzung gebracht werden oder über einen Spacer an das Polysaccharid gebunden werden.

Die im Sinne der Erfindung eingesetzten Farbstoffkomponenten der Konjugate haben ein Absorptionsmaximum im Bereich zwischen 500 und 1300 nm, vorzugsweise zwischen 600 und 900 nm. Hierfür eignen sich vor allem Phthalocyanin-, Phenazin-, Phenothiazin-, Phenoxazin-, Rhodamin-, Azo-, Triphenylmethan und Cyaninfarbstoffe und deren Derivate. Repräsentative Vertreter einzelner Farbstoffe sind z.B. Triphenylmethane, Indocyanine, Oxazine und Rhodamine.

Gegenstand der Erfindung sind daher Verbindungen der allgemeinen Formel I

F-X-(Spacer)ₙ-Y-E-(CH₂)ₘ-PS (I),

in der
F = eine Farbstoffkomponente mit einem Absorptionsmaximum zwischen 500 und 1300 nm, vorzugsweise zwischen 600 und 900 nm,
n = 0 oder 1,
m = 0 oder 1,
X für den Fall, daß n = 0 ist, =
-CO, -NH-C=O, -NH-C=S, -NH-C=NH, -CONH-C=S,
X für den Fall, daß n = 1 ist, =
NH, -CONH, -SO₂NH, -NHCONH, -NHCSNH,

Spacer = eine C₂-C₆ Alkylengruppe oder die Gruppe 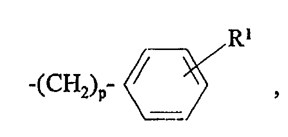 wobei
p = 0 - 4 und R₁ = Wasserstoff oder die Sulfonsäuregruppe sein kann,
Y = Valenzstrich, -CH₂, -NHCOCH₂, -NH-C=O, -NH-C=S, -NH-C=NH,
E = O oder NH
und PS = ein Polyfructosan darstellt, wobei im Falle, daß E = O und m = 0 ist, das Sauerstoffatom , über das das Polysaccharid gebunden ist, Teil dieses Polysaccharids ist, und im Falle, daß E = NH ist, es sich um ein aminiertes bzw. aminoalkyliertes Polyfructosan handelt.

Bevorzugt im Sinne der Erfindung sind vor allem Verbindungen, in denen X = -CO, -NH, -CONH, -SO₂NH, -NH-C=O, -NH-C=S,

Spacer = eine C₂ - C₆-Alkylengruppe oder die Gruppe 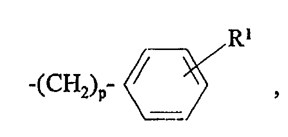 wobei
p bevorzugt 0 oder 1 ist und R₁ Wasserstoff darstellt,
n = 0 oder 1,
Y = Valenzstrich, -CH₂, -NH-COCH₂, -NH-C=O,
m = 0 oder 1,
E = O oder NH
und PS vorzugsweise der Sinistrin-, Inulin-Rest oder ein aminiertes oder aminoalkyliertes Derivat von Sinistrin oder Inulin darstellen.

Insbesondere sind hervorzuheben Verbindungen der allgemeinen Formel I, in denen F einen Triphenylmethan-, Indocyanin-, Oxazine- oder Rhodaminfarbstoff, X die -CO-, -CONH-Gruppe, Spacer einen C₂ - C₄-Rest, n = 0 oder 1, m = 0 oder 1, Y = Valenzstrich, CH₂ oder-NHCOCH₂ und E = O oder NH darstellen und es sich bei dem Polysaccharid um Sinistrin , Inulin oder ein aminiertes oder aminoalkyliertes Derivat von Sinistrin oder Inulin handelt.

Die Polysaccharide können ein- oder mehrfach mit den Farbstoffen reagiert haben und somit unterschiedliche Belegungsgrade aufweisen.

Zur besseren Wasserlöslichkeit können die Verbindungen der allgemeinen Formel I gegebenenfalls nachträglich an der Polysaccharideinheit derivatisiert werden. Hierzu eignen sich insbesondere Derivate mit 2-Hydroxypropyl- bzw. Carboxymethylgruppierungen an der Polysaccharideinheit. Zur besseren Wasserlöslichkeit aber auch zum Neutralisieren überschüssiger Aminogruppen im modifizierten Polysaccharid kann man Verbindungen der allgemeinen Formel I auch mit Sultonen wie z.B. dem 1.4-Butansulton umsetzen. Diese Substanzen sind ebenfalls Gegenstand dieser Erfindung.

Substanzen der allgemeinen Formel I lassen sich nach allgemein bekannten Verfahren herstellen, vorzugsweise indem man
1. ein Farbstoffderivat der allgemeinen Formel II

   F-Z (II),

   in der
   F eine Farbstoffkomponente mit einem Absorptionsmaximum von 500 - 1300 nm,
   Z eine -NCO, -NCS, -NC=NH, - CONCS oder die Gruppe G - Spacer - NCO, G -Spacer -NCS, G -Spacer- NCNH
   darstellt,
   wobei G = NH, -CONH, -SO₂NH, -NH-CO-NH, -NH-CS-NH bedeutet und Spacer die oben angegebenen Bedeutungen hat,
   mit einem Polyfructosan der allgemeinen Formel III

   HO- PS (III),

   in der PS-OH eines der oben aufgeführten Polyfructosane darstellt, umsetzt
   oder
2. ein Farbstoffderivat der allgemeinen Formel IV

   F-D (IV),

   in der D eine NH₂ oder eine -G-Spacer-NH₂-Gruppe darstellt, wobei F, G und Spacer die oben angegebenen Bedeutungen haben, mit einem Polyfructosan - derivat der allgemeinen Formel V 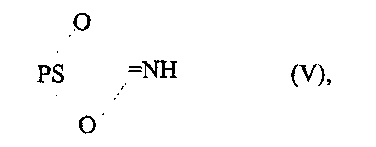 in der PS die oben angegebene Bedeutung hat,
   umsetzt
   oder
3. ein Farbstoffderivat der allgemeinen Formel IV

   F-D (IV),

   in der F und D die oben angegebenen Bedeutungen haben, mit einem Polyfructosanderivat der allgemeinen Formel VI

   PS-O-CH₂-COOH (VI),

   in der PS die oben angegebene Bedeutung hat,
   umsetzt
   oder
4. ein Farbstoffderivat der allgemeinen Formel VII

   F-A (VII),

   in der F die oben angegebene Bedeutung hat und A eine Aktivestergruppierung wie z.B. N-Hydroxisuccinimidester oder N-Hydroxi-benzotriazolester, eine Carbon- oder Sulfonsäurechloridgruppe bedeuten, mit einem Amin der allgemeinen Formel VIIIa bzw VIIIb

   H₂N-Spacer-Y-O-PS (VIIIa)

   H₂N-(CH₂)ₘ-PS (VIIIb),

   in der Spacer und PS die oben angegebenen Bedeutungen haben, Y = Valenzstrich, -CH₂, -NHCOCH₂, -NH-C=O, -NH-C=S, -NH-C=NH, m = 0 oder 1 sein sollen, umsetzt.

Die Herstellung eines Isocyanatderivates der allgemeinen Formel II geschieht in-situ durch thermische Zersetzung des entsprechenden Carbonsäureazids wie in Chem. Pharm. Bull. 33, 1164 (1985) beschrieben. Die Umsetzung eines Farbstoffisocyanatderivates der allgemeinen Formel II mit einem Polysaccharid der allgemeinen Formel III geschieht z.B. analog Makromol. Chemie 121, 18 (1968).

Isothiocyanate der allgemeinen Formel II lassen sich aus Aminen der allgemeinen Formel F-NH₂, in der F die oben angegebene Bedeutung hat, analog Chem. Ber. 63, 888 (1930), J. Chem. Soc. 125, 1702 (1924) oder Chem. Ber. 86, 314 (1953) herstellen. Deren Umsetzung mit Polysacchariden der allgemeinen Formel III läßt sich analog Chem. Zentralblatt 1910, 910 oder Am.Chem. J. 22, 464 bzw. J. Am. Chem. Soc. 65, 900 (1943) durchführen.

Amine der allgemeinen Formel IV, in der D eine -G-Spacer-NH₂-Gruppe darstellt, sind literaturbekannt oder werden nach an sich bekannten Methoden dargestellt. So kann man z.B. im Falle von G = CONH bzw. SO₂NH eine Carbonsäure bzw. Sulfonsäure der allgemeinen Formel F-CO₂H bzw. F-SO₃H, in der F die oben angegebene Bedeutung hat, über den entsprechenden Aktivester (Reaktionsführung s. unten) oder das entsprechende Säurechlorid mit einem Diamin der allgemeinen Formel H₂N-Spacer-NH₂, in der Spacer die oben angegebene Bedeutung hat, unter geeigneten Reaktionsbedingungen zu einem Amid der allgemeinen Formel IV umsetzen (s. hierzu J. Med. Chem. 27, 1481(1984), J. Prakt. Chem. 130, 293 (1931), J.Med. Chem. 34, 73 (1991) und Liebigs Ann. Chem. 1988, 787).

Polysaccharidderivate der allgemeinen Formel V werden durch Umsetzung des Polysaccharids mit Bromcyan hergestellt. Solche Verbindungen sind z.B. in Nature 214, 1302 (1967) oder Eur.J.Biochem. 18, 351 (1971) beschrieben. Die weitere Umsetzung mit Aminderivaten der allgemeinen Formel IV zu Verbindungen der allgemeinen Formel I ist u.a. in Carbohydrate Res. 20, 1 (1971) beschrieben.

Polyfructosanessigsäuren der allgemeinen Formel VI sind u.a. in Methods Carbohydr. Chem. 6, 384 (1972) und Khim. Prir. Soedin 1969, 525 beschrieben.

Polyfructosancarbonsaurederivate der allgemeinen Formel VI werden in aktivierter Form mit Aminen der allgemeinen Formel IV zur Reaktion gebracht. Als aktive Form eignen sich in diesem Fall vor allem Aktivester. So wird eine Carbonsäure der allgemeinen Formel VI mit Alkoholen wie N-Hydroxy-succinimid oder N-Hydroxybenzotriazol in Gegenwart eines wasserabspaltenden Agenzes wie Dicyclohexylcarbodiimid in inerten Lösungsmittel wie z.B. Essigsäureethylester, Dichlormethan, Tetrahydrofuran oder N,N-Dimethylformamid zur Reaktion gebracht. Der so hergestellte Aktivester wird in der Regel direkt in die weitere Reaktion unter Zusatz von organischen Basen wie Triethylamin eingesetzt. Man kann die Reaktion aber auch sehr gut in Wasser durchführen, was in einigen Fällen aus Löslichkeitsgründen vorzuziehen ist. In diesem Fall setzt man als Carbodiimid vorzugsweise das N-Ethyl-N'(3-trimethylammonio-propyl) carbodiimid-jodid ein. Die Reaktion läßt sich jedoch auch ohne Zusatz von z.B. N-Hydroxy-succinimid mit N-Ethyl-N'(3-dimethylaminopropyl) carbodiimid durchführen.

Die Umsetzung eines Farbstoffderivates der allgemeinen Formel VII mit Aminen der allgemeinen Formel VIIIa bzw. VIIIb geschieht analog dem im vorherigen Absatz beschriebenen Verfahren oder analog S. 10. Verbindungen der allgemeinen Formel VIIIa sind teilweise literaturbeschrieben (Starch 48(5), 191 (1996); J. Nucl. Med. 27, 513 (1986)). Sie lassen sich aber auch durch Umsetzung einer Polyfructosanessigsäure der allgemeinen Formel VI mit einem monogeschützten Alkylendiamin analog dem im vorherigen Absatz beschriebenen Verfahren zur Reaktion bringen. Als Schutzgruppe eignet sich in diesem Falle vor allem die Carbobenzoxygruppe, die anschließend leicht katalytisch mit Wasserstoff abgespalten werden kann.
Man kann Amine der allgemeinen Formel VIIIa auch dadurch herstellen, daß man das Polyfructosan in einem inerten Lösungsmittel wie z.B. N,N-Dimethylformamid mit Natriumhydrid deprotoniert, anschließend mit z.B. p-Cyan-benzylbromid alkyliert und das so gewonnene Nitril in Gegenwart von Raney-Nickel oder Raney-Kobalt unter Zusatz von konzentriertem Ammoniak in einem Methanol-Wasser-Gemisch hydriert. Die Alkylierung kann z.B. auch mit 2-Bromethylamin vorgenommen werden, wodurch man ein O-Aminoethyl-polysaccharid erhält ( J. Nucl. Chem. 27, 513 (1986)). Amine der allgemeinen Formel VIIIb kann man auf folgende Weise herstellen. Durch die Umsetzung eines Polyfructosans mit p-Toluolsulfochlorid in einem inerten Lösungsmittel wie z.B. N,N-Dimethylformamid in Gegenwart einer Base erhält man ein p-Toluolsulfonat des Polyfructosans, wobei der Belegungsgrad von der eingesetzten Menge des p-Toluolsulfochlorids abhängt. Reaktion dieses Sulfonsäureesters mit Natriumazid in einem Lösungsmittel wie z.B. N,N-Dimethylformamid bei Temperaturen von 80 - 100°Celsius ergibt das entsprechende Azid, aus dem durch katalytische Reduktion mit Wasserstoff ein Polyfructosan entsteht, das direkt am Kohlenwasserstoffgerüst eine Aminogruppe trägt. Setzt man den gerade beschriebenen Sulfonsäureester mit Natriumcyanid in z.B. N,N-Dimethylformamid bei Temperaturen zwischen 80 - 100°Celsius um, erhält man das entsprechende Nitril, das man durch katalytische Hydrierung mit Raney-Nickel oder Raney-Kobalt in einem Methanol-Wasser-Gemisch in Gegenwart von konzentriertem Ammoniak zu dem gewünschten primären Amin reduzieren kann. In diesem Fall trägt das Kohlenwasserstoffgerüst eine Aminomethylgruppe.

Weitere Verfahren zur Herstellung von aminierten bzw. aminoalkylierten Polysacchariden sind in der Literatur beschrieben (s. z.B. J. Med. Chem. 31, 898 (1988)). So kann man unter geeigneten Reaktionsbedingungen eine oder auch mehrere Hydroxylgruppen in einem Polysaccharid mit Natriumperjodat zum Aldehyd oxydieren, der dann in Gegenwart eines Amins mit Cyanoborhydrid zum alkylierten Amin reduziert wird. Als Aminkomponente werden in diesem Fall bevorzugt monogeschützte Alkylendiamine eingesetzt, wobei als Schutzgruppe vor allem die Carbobenzoxygruppe Verwendung findet. Diese Schutzgruppe wird dann katalytisch unter allgemein bekannten Bedingungen in Wasser als Lösungsmittel abhydriert.

Die nachträgliche Überführung von Verbindungen der allgemeinen Formel I in wasserlöslichere Substanzen durch Umsetzung mit Chloressigsäure bzw. Propenoxyd läßt sich analog literaturbekannter Verfahren durchführen. So ist die Umsetzung eines Polysaccharids mit Chloressigsäure u.a. in Methods Carbohydr. Chem. 6, 384 (1972) oder Makromol. Chem. 122, 271 (1969) und die Reaktion eines Polysaccharids mit Propenoxyd in "Starch Chemistry and Technology", Academic Press New York (1984), R.L. Whistler et al. beschrieben.

Die erfindungsgemäßen Verbindungen der allgemeinen Formel I werden in der Regel intravenös verabreicht. Hierzu werden 10 - 25% ige isotonische, wässrige Lösungen eingesetzt, die die bei Injektionen üblichen Zusätze wie Stabilisierungsmittel und Puffer enthalten. Als Puffer werden vorzugsweise Natrium- oder Kaliumphosphatpuffer verwendet, als Stabilisierungsmittel können gegebenenfalls Antioxidantien zum Einsatz kommen. Um eine isotone Lösung zu erhalten, wird Natriumchlorid oder Mannit zugesetzt. Der pH-Wert der Lösung liegt zwischen 6,5 und 8, vorzugsweise zwischen pH = 7 und 7,5. Die erfindungsgemäßen Verbindungen der allgemeinen Formel I werden einmalig in einer Menge von 0,1 - 5 g, vorzugsweise 0,2 - 2 g appliziert.

Die folgenden Beispiele zeigen einige Verfahrensvarianten, die zur Synthese der erfindungsgemäßen Verbindungen verwendet werden können. Die Struktur der Verbindungen wurde durch ¹H und ¹³C-Kernresonanzspektroskopie gesichert. Weiterhin wurden die hergestellten Konjugate durch Gelpermeationschromatographie überprüft (Ultrahydrogel™ 250, 7,8 x 300 mm Säule von der Firma Waters; Fluß: 0,5 ml/min; Wasser). Hierbei wurden die λₘₐₓ-Werte der Polysaccharid- und der Farbstoffkomponente zum selben Zeitpunkt t ermittelt.

### Zur Kupplung eingesetzte Farbstoffe

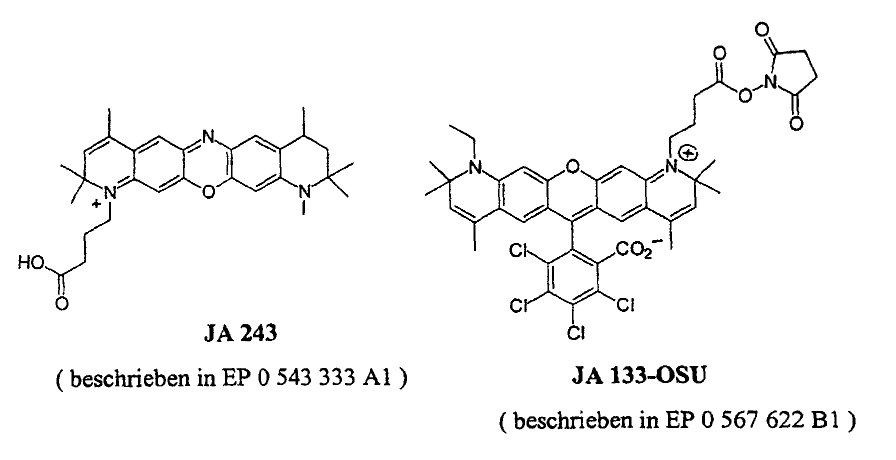

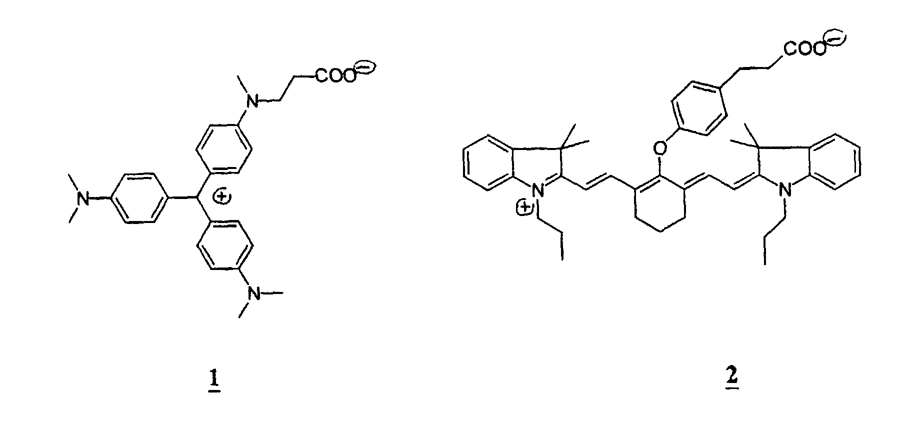

### Synthese von 1

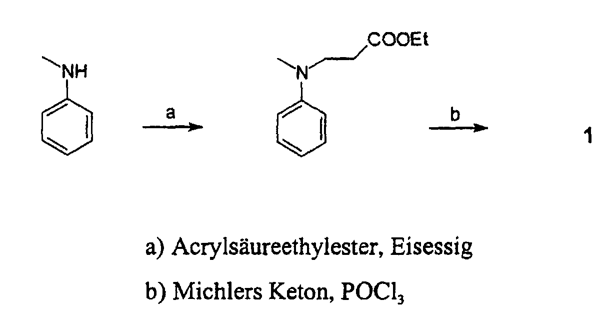

***N*-(2-Ethoxycarbonyl-ethyl)-*N*-methyl-anilin.** Analogvorschrift: *Organikum,* **1988**, 17. Aufl., 334.

**1**. 25.9 g *N*-(2-Ethoxycarbonyl-ethyl)-*N*-methyl-anilin (125 mmol) wurden mit 6.71 g (25 mmol) Michlers Keton und 9.20 g Phosphoroxychlorid (60 mmol) 3 Stunden im Wasserbad erhitzt. Der Ansatz wurde über Nacht bei Raumtemperatur gerührt, mit 500 ml Waser versetzt und mit Ätznatron (400 mmol in 100 ml Wasser) alkalisch gestellt. Nun wurden mit Wasserdampf zunächst 100 ml Destillat ausgetrieben. Nach der Zugabe von 10 g Ätznatron in 50 ml Wasser wurden weitere 150 ml im Wasserdampfstrom abdestilliert. Der braune Niederschlag wurde abgesaugt, mit Wasser nachgewaschen, mit 300 ml 1 N Salzsäure bei 90 °C 20 Minuten ausgerührt und heiß filtriert. Das rohe Produkt wurde ausgesalzt, aus Wasser umkristailisiert (scheidet sich zunächst als Öl ab) und über Calciumchlorid im Vakuum getrocknet.
Ausbeute: 8.55 g (73 %) metallisch grüner Feststoff.

**1-Hydroxysuccinimidester**: 1.0 g **1** (1.72 mmol, berechnet auf **1**xHCl) wurde bei 0°C in 25 ml getrocknetem Acetonitril gelöst und mit 375 mg (2.6 mmol) N-Hydroxy-succinimid, sowie 670 mg (2.6 mmol) Dicyclohexyl-carbodiimid versetzt. Nach 30 Minuten wurde das Kühlbad entfernt und der Ansatz 2.5 Stunden bei Raumtemperatur gerührt. Die so erhaltene Lösung wurde filtriert und roh weiterverarbeitet.

### Synthese von 2

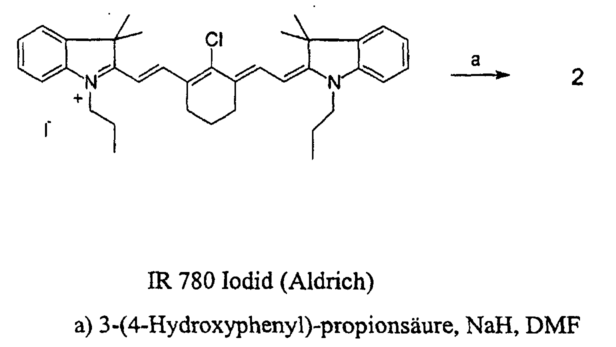

**2.** Zu 6.9 mmol Natriumhydrid in 50 ml Dimethylformamid wurden bei 0 °C 573 mg (3.45 mmol) 3-(4-Hydroxyphenyl)-propionsäure gegeben. Die heterogene Mischung wurde 30 Minuten bei Raumtemperatur gerührt, anschließend mit 2.00 g (3.00 mmol) festem Aldrich IR 780-Iodid versetzt und über Nacht bei Raumtemperatur gerührt. Die gesamte Reaktionsmischung wurde auf eine mit Kieselgel/Chloroform gepackte Säule gegeben und mit einem Laufmittelgradienten von Chloroform nach Chloroform : Methanol = 70 30 chromatographiert.
Ausbeute: 1.45 g (61 %), tiefgrüner, metallisch glänzender Feststoff.

**2-Hydroxysuccinimidester :** 1.60 g (2.39 mmol) **2** in 50 ml trockenem Acetonitril wurden mit 413 mg (3.59 mmol) N-Hydroxysuccinimid versetzt und im Eisbad auf 0 °C gekühlt. 741 mg (3.59 mmol) Dicyclohexylcarbodiimid wurden zugegeben und der Ansatz bei auftauendem Eisbad über Nacht gerührt. Die Reaktionsmischung wurde filtriert, vom Lösungsmittel befreit und der Rückstand im Vakuum getrocknet. Ausbeute: 1.43 g rohes Produkt, ohne Reinigung weiterverarbeitet.

### Beispiel 1

### Konjugat aus 1 mit O-Aminoethylinulin

1.00 g O-Aminoethylinulin (Mᵣ ca. 175ₙ, 5.71 mmol; Gehalt an freien Aminofunktionen ca. 0.57 mmol) wurden in 20 ml Wasser gelöst und innerhalb von 3 Minuten mit einer Lösung von **1**-Hydroxysuccinimidester (10 ml, 0.69 mol) in Acetonitril (Gehalt bez. auf unaktivierte Säure = 69 µmol/ml) versetzt. Nach 3 Stunden und nach 5 Stunden wurde jeweils mit weiteren 5 ml **1**-Hydroxysuccinimidester-Lösung (0.35 mmol) versetzt und der Ansatz 2 Tage bei Raumtemperatur gerührt. Das Lösungsmittel wurde unter vermindertem Druck abdestilliert, der Rückstand im Vakuum getrocknet und in 150 ml Methanol aufgenommen. Nach Zugabe von 150 ml Aceton fiel das rohe Produkt aus. Es wurde abzentrifugiert, zweimal in Aceton aufgeschlämmt und erneut abzentrifugiert. Zur Reinigung wurde in wenig Wasser aufgenommen, filtriert und in zwei Portionen über eine Gelsäule (*BioRad* Bio-Gel, P-2 fine, Säule 2.5x60 cm, Fluß ca. 1 ml/min Eluens: destilliertes Wasser) chromatographiert.
Ausbeute:630 mg, tiefvioletter Feststoff; λₘₐₓ bei 204 und 590 nm nach 12 Minuten

### Aminoethylierung von Inulin

### A Umsetzung mit Bromethylamin

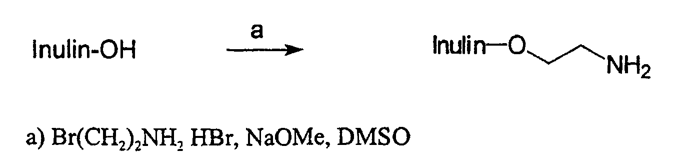

Lit.: J. G. McAfee, F. D. Thomas, G. Subramanian, R. D. Schneider, B. Lyons, M. Roskopf, C. Zapf-Longo, D. Whaley, *J. Nucl. Chem.* **1986**, 27, 513-520.

### B Synthese über N-Carbobenzoxy-bromethylamin

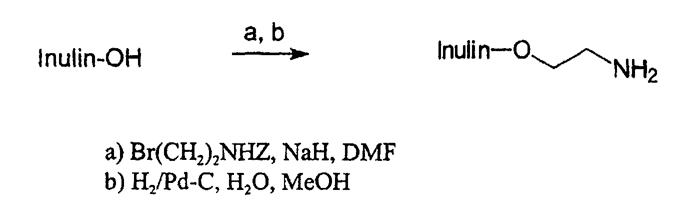

**O-(Aminoethyl)inulin.** Bei ca. 30 °C wurde Inulin (2.00 g ,12.3 mMol, bezogen auf Fructoseeinheiten) in getrocknetem Dimethylformamid (30 ml) gelöst und bei Raumtemperatur mit Natriumhydrid (2.22 g, angefeuchtet mit ca. 40 % Öl, 55.5 mMol) versetzt. Nach 30 Minuten wurde *N*-Carbobenzoxy-2-brom-ethylamin (4.76 g, 18.4 mMol) zugegeben und auf 100°C erwärmt. Innerhalb von 15 Minuten wurden weitere 4.76 g *N*-Carbobenzoxy-2-brom-ethylamin zugegeben und die Reaktionsmischung 2.5 Stunden bei 100°C und über Nacht bei Raumtemperatur gerührt. Unter Kühlung im Eisbad, wurde vorsichtig mit dem gleichen Volumen Wasser versetzt und Ammoniumchlorid-Lösung (1.07 g in 5 ml Wasser) zugegeben. Das Lösungsmittel wurde bei vermindertem Druck abdestilliert, der Rückstand im Vakuum getrocknet und in 200 ml Wasser aufgenommen. Es wurde mit Diethylether ausgeschüttelt und die wäßrige Phase auf ca. 5 ml eingeengt (ein eventuell auftretender Niederschlag wird abfiltriert).
Die Reinigung erfolgte über eine präparative Gelpermeationschromatographie. *BioRad* Bio-Gel, P-2 fine. Säule 2.5x60 cm, Fluß ca. 1 ml/min Eluens: destilliertes-Wasser.
Ausbeute: 1.70 g
Abspaltung der Carbobenzoxy-Schutzgruppe:
150 mg *N*-Carbobenzoxy-aminoethylinulin wurden in 10 ml Wasser aufgenommen, mit 1 ml Methanol und 75 mg Palladium-Kohle (Chempur, 10 % Pd auf C) versetzt und 2 Tage bei Raumtemperatur unter Normaldruck hydriert. Der Katalysator wurde abfiltriert, das Filtrat vom Lösungsmittel befreit und der Rückstand im Vakuum getrocknet.
Ausbeute: 111 mg

### Beispiel 2

### Konjugat aus JA 243 und O-Aminoethylinulin

20 mg O-Aminoethylinulin (Mᵣ ca. 175ₙ, 110 µmol) wurden in 0.5 ml Wasser gelöst und auf 50 °C erwärmt. Hierzu wurde in zwei Portionen im Abstand einer Stunde eine Lösung von 10 mg **JA 243**-Hydroxysuccinimidester (17.5 µmol; beschrieben in EP 0 543 333 A1) in 1 ml Acetonitril getropft. Nach 4 Stunden wurden weitere 3 mg Farbstoff-Aktivester (5.2 µmol) fest zugegeben und der Ansatz bei Raumtemperatur 20 Stunden gerührt. Das Lösungsmittel wurde bei vermindertem Druck abdestilliert und der Rückstand im Vacuum getrocknet. Die Reinigung erfolgte durch Gelpermeationschromatographie (*BioRad* Bio-Gel, P-2 extra fine, Säule 1x20 cm, Eluens: destilliertes-Wasser)
Ausbeute: 13 mg tiefblauer Feststoff; λₘₐₓ bei 204 und 660 nm nach 12,5 Minuten

### Beispiel 3

### Konjugat aus JA 133 und O-Aminoethylinuli

750 mg O-Aminoethylinlin ( Mᵣ ca. 175ₙ, 4.3 mmol) wurden in 7 ml Wasser gelöst. Hierzu wurden tropfenweise 4 ml Acetonitril gegeben. Die klare Lösung wurde auf 40 °C erwärmt und mit 210 mg (0.25 mmol) festem **JA 133-OSu** versetzt. Nach 2 Stunden wurden 100 µl *N*-Methylmorpholin und 2 ml Acetonitril zugegeben. Der Ansatz wurde 20 Stunden bei Raumtemperatur gerührt, dabei wurden nach 3 Stunden weitere 130 mg (0.15 mmol) **JA 133-OSu** zugegeben. Nach 5 Stunden bei 45 °C wurden erneut 100 mg (0.12 mmol) **JA 133-OSu** zugegeben und über Nacht bei Raumtemperatur gerührt. Zur Aufarbeitung wurde mit Wasser versetzt und viermal mit Chloroform ausgeschüttelt. Die wäßrige Lösung wurde 3 Stunden bei 35 °C gehalten, eingeengt und über eine Gelsäule (*BioRad* Bio-Gel, P-2 extra fine, Säule 2.5x35 cm, Fluß ca. 0.2 ml/min Eluens: destilliertes-Wasser) chromatographiert.
Ausbeute: 415 mg; λₘₐₓ bei 204 und 621 nm nach 16 Minuten.

### Beispiel 4

### Konjugat aus 2 mit O-(3-Aminopropyl)inulin

Bei Raumtemperatur wurden 1,00 g O-( 3-Aminopropyl)inulin ( Firma Cosun Industrial Inulin Derivatives) (Carbonat/Hydrogencarbonat-Gemisch, Belegung mit Aminopropylfünktionen/Fructoseeinheit: 0,60) in 20 ml Wasser gelöst und tropfenweise mit 12,5 ml Acetonitril versetzt. Zu dieser Lösung wurden zunächst 1,14 g und nach 1 Stunde weitere 0,1 g **2**-Hydroxysuccinimidester fest zugegeben. Die Reaktionsmischung wurde über Nacht gerührt, mit 200 ml Wasser versetzt und dreimal mit Chloroform ausgeschüttelt. Das Produkt wurde über eine Gelsäule (BioRad Bio-Gel, P-2 extra fine, Säule 2,5 x 20 cm, Eluens: destilliertes Wasser) vorgereinigt und anschließend chromatographiert (gleiche Bedingungen, Säule 2,5 x 40 cm)
Ausbeute: 600 mg

### Beispiel 5

### Konjugat aus JA 133 und O-(3-Aminopropyl)inulin

Bei Raumtemperatur wurden 700 mg O-(3-Aminopropyl)inulin in 14 ml Wasser gelöst und tropfenweise mit Acetonitril versetzt, bis eine Trübung bestehen blieb. Es wurden 0,5 ml Wasser zugegeben und die nun klare Lösung mit 690 mg (0,821 mmol) festem **JA 133-OSU** versetzt. Der Ansatz wurde über Nacht bei Raumtemperatur gerührt, mit 200 ml Wasser versetzt und dreimal mit Chloroform ausgeschüttelt. Die wässrige Phase wurde einrotiert, in wenig Wasser aufgenommen und über eine Gelsäule ( BioRad Bio-Gel, P-2 extra fine, Säule 2,5 x 35 cm, Eluens: destilliertes Wasser) chromatographiert.
Ausbeute: 550 mg

### Beispiel 6

### Konjugat aus JA 133 mit 3a

4.4 mg 3a in 90 µl Wasser und 30 µl Acetonitril wurden mit 8 µl *N*-Ethylmorpholin versetzt. Hierzu wurde eine Lösung von 4 mg **JA 133-OSu** in 80 µl Acetonitril und 240 µl Wasser gegeben. Nach 6 Tagen wurden im Abstand von 2.5 Stunden jeweils 3 mg **JA 133-OSU** in 60 µl Acetonitril und 180 µl Wasser zugesetzt. Nach 1 weiteren Tag wurde vom Lösungsmittel befreit, in 1 ml Wasser aufgenommen und über eine Gelsäule (*BioRad* Bio-Gel, P-2 Extra Fine. Säule 2x13 cm, Eluens: destilliertes Wasser) chromatographiert.
Ausbeute: 1.5 mg; λₘₐₓ bei 204 und 621 nm.

### Synthese von 3a

**Carboxymethylinulin.** Lit.: D. L. Verraest, J. A. Peters, J. G. Batelaan, H. v. Bekkum, *Carbohydrate Research,* **1995**, *271*, 101-112. 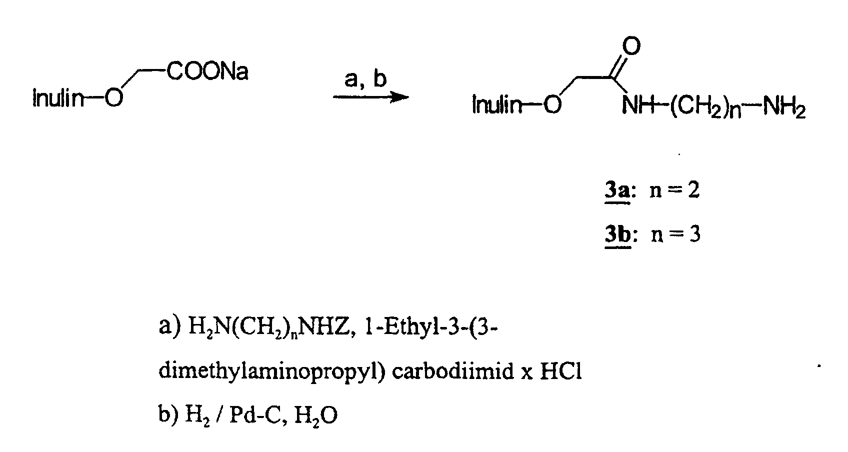 **n = 2**. 1.91 g (8.67 mmol) Carboxymethylinulin wurden in 19 ml 2-Morpholinoethansulfonsäure-Natronlauge-Puffer ( pH 4.75) gelöst. Bei Raumtemperatur wurden 3.32 g (17.3 mmol) 1-Ethyl-3-(3-dimethylaminopropyl)carbodiimid-hydrochlorid und 2.00 g (8.67 mmol) *N*-Carbobenzoxy-diaminoethan-hydrochlorid (FLUKA) zugegeben. Nach 1 Stunde wurde kurz auf 40° C erwärmt und über Nacht bei Raumtemperatur gerührt. Es wurden noch 2.39 g (12.4 mmol) 1-Ethyl-3-(3-dimethylaminopropyl)carbodiimid-hydrochlorid zugegeben und weitere 5 Stunden gerührt. Die Lösung wurde mit Natronlauge auf pH 11 eingestellt, der ausgefallene Niederschlag abgetrennt, die wäßrige Phase dreimal mit 80 ml Dichlormethan ausgeschüttelt und gegen wäßrige Kochsalzlösung (toluolhaltig) dialysiert.
Ausbeute: 870 mg

### Abspaltung der Schutzgruppe:

300 mg des N-Carbobenzoxy-geschützten Aminoinulinderivates wurden in 5 ml Wasser gelöst, mit 50 mg Katalysator (10% Palladium auf C) versetzt und 5 Stunden bei 40 mbar hydriert. Die Lösung wurde filtriert und lyophilisiert.
Ausbeute: 143 mg.

### Beispiel 7

### Konjugat aus JA 243 mit 3a

6 mg **3a** in 250 µl Wasser und 250 µl Acetonitril wurden mit 11 µl *N*-Ethylmorpholin versetzt. Zu der nun klaren Lösung wurden 3.7 mg und nach 3 Stunden 2.7 mg **JA 243**-Hydroxysuccinimidester (beschrieben in EP 0 543 333 A1) in jeweils 50 µl Acetonitril und 50 µl Wasser getropft. Es wurden 8.2 µl *N*-Ethylmorpholin zugegeben und über Nacht gerührt. Nach dem Entfernen des Lösungsmittels konnte das Produkt durch eine präparative Gelpermeationschromatographie (*BioRad* Bio-Gel, P-2 extra fine, Säule 2x30 cm, Eluens: destilliertes Wasser) isoliert werden.
Ausbeute: 1,8 mg; λₘₐₓ bei 204 und 660 nm.

### Beispiel 8

### Konjugat aus 1 mit 3a

Eine Lösung von 600 mg **3a** in 25 ml Wasser wurde mit 1 ml *N*-Ethylmorpholin versetzt und zu einer Lösung von 2.15 mmol **1**-Hydroxysuccinimidester in 25 ml Acetonitril gegeben. Der Ansatz wurde über Nacht gerührt und mit weiteren 2.15 mol **1**-Hydroxysuccinimidester in 25 ml Acetonitril versetzt. Nach 5 Stunden wurden 100 ml Wasser zugegeben, auf 50 °C erwärmt und über Nacht bei Raumtemperatur stehen gelassen. Das Lösungsmittel wurde entfernt, der Rückstand mit 100 ml Methanol ausgerührt und zentrifugiert. Der Überstand wurde verworfen, das rohe Produkt in 20 ml Wasser aufgenommen und durch präparative Gelpermeationschromatographie (Bedingungen s. Beispiel 7) gereinigt.
Ausbeute: 420 mg; λₘₐₓ bei 204 und 590 nm.

### Beispiel 9

### Konjugat aus JA 133 mit 3b

30 mg **3b** wurden in einer Mischung von 0.3 ml Wasser und 0.2 ml Acetonitril gelöst und mit 38 µl *N*-Ethylmorpholin versetzt. Die so erhaltene Lösung wurde bei Raumtemperatur zu 5 mg (6 µmol) **JA 133-OSu** in 60 µl Wasser und 40 µl Acetonitril getropft. Nach 2.5 Stunden wurde die Reaktionsmischung zu weiteren 5 mg (6 µmol) **JA 133-OSu** in 60 µl Wasser und 40 µl Acetonitril getropft und über Nacht gerührt. Das Lösungsmittel wurde entfernt und der Rückstand im Vakuum getrocknet. Es wurde in 0.2 ml Wasser aufgenommen und durch Gelchromatographie gereinigt (*BioRad* Bio-Gel, P-2 extra fine, Säule 2x30 cm, Eluens: destilliertes Wasser).
Ausbeute: 25 mg; λₘₐₓ bei 204 und 621 nm.

### Synthese von 3b

208mg (0.95 mmol) Carboxymethylinulin wurden in 2 ml 0.2 M 2-Morpholinoethansulfonsäure-Natronlauge-Puffer ( pH 4.75) gelöst und mit 362 mg (1.89 mmol) 1-Ethyl-3-(3-dimethylaminopropyl) carbodiimid-hydrochlorid versetzt. Der pH wurde mit Salzsäure auf 4.75 gebracht, 231 mg (0.95 mmol) *N*-Carbobenzoxy-diaminoethan-hydrochlorid (FLUKA) zugegeben und der Ansatz über Nacht bei Raumtemperatur gerührt. Mit Natronlauge wurde der pH erneut auf 4.75 eingestellt und nach weiteren 4 Stunden neutralisiert. Die Produktlösung wurde mit Dichlormethan extrahiert und 2 Tage gegen Wasser dialysiert.
Ausbeute: 126 mg.

### Abspaltung der Schutzgruppe:

Vorgehensweise wie bei n = 2. Aus 102 mg *N*-Carbobenzoxy-amin wurden 46 mg freies Amin erhalten.

In der folgenden Tabelle werden die voranstehend beschriebenen Verbindungen sowie weitere bevorzugte Verbindungen aufgelistet.

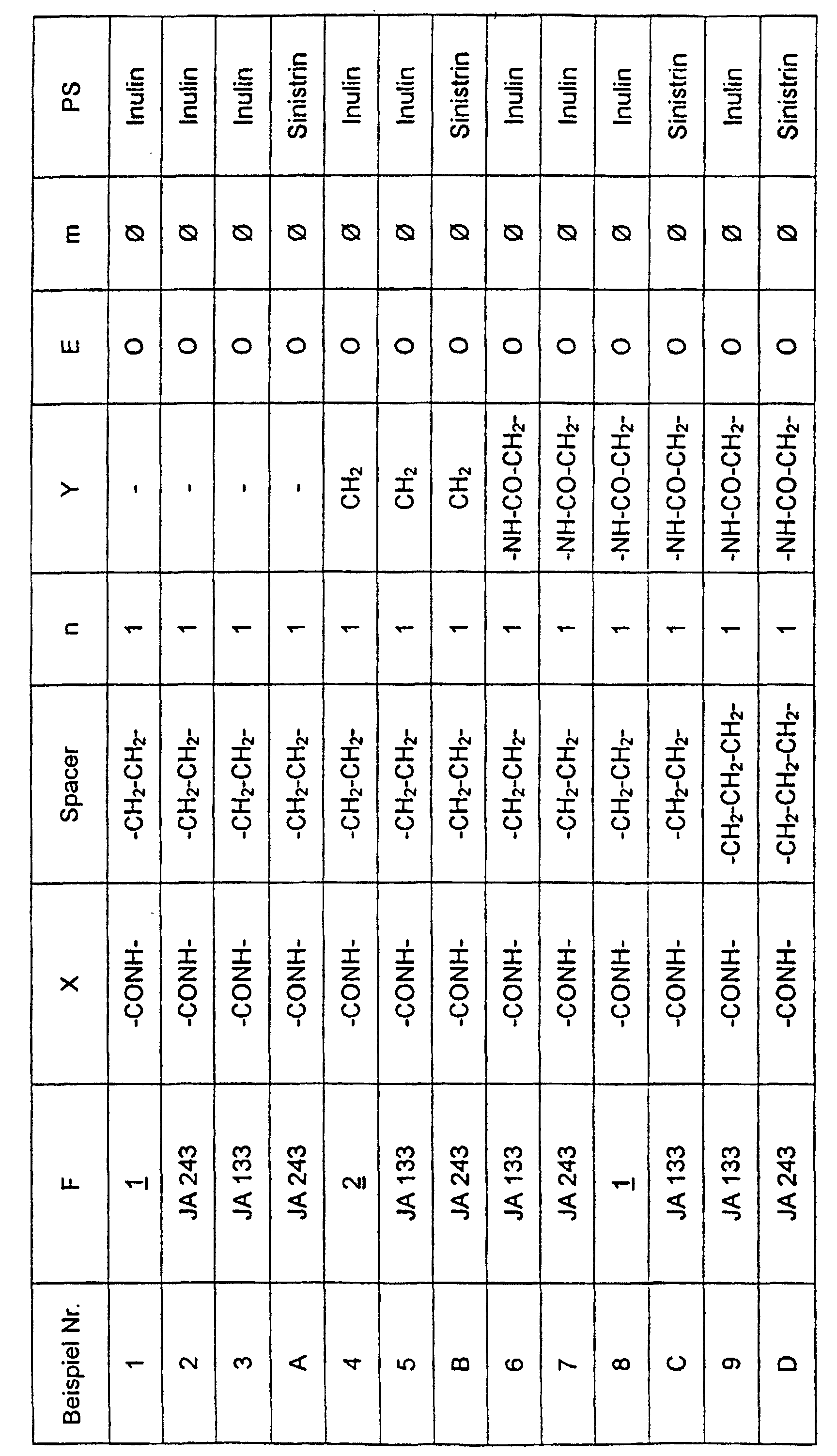

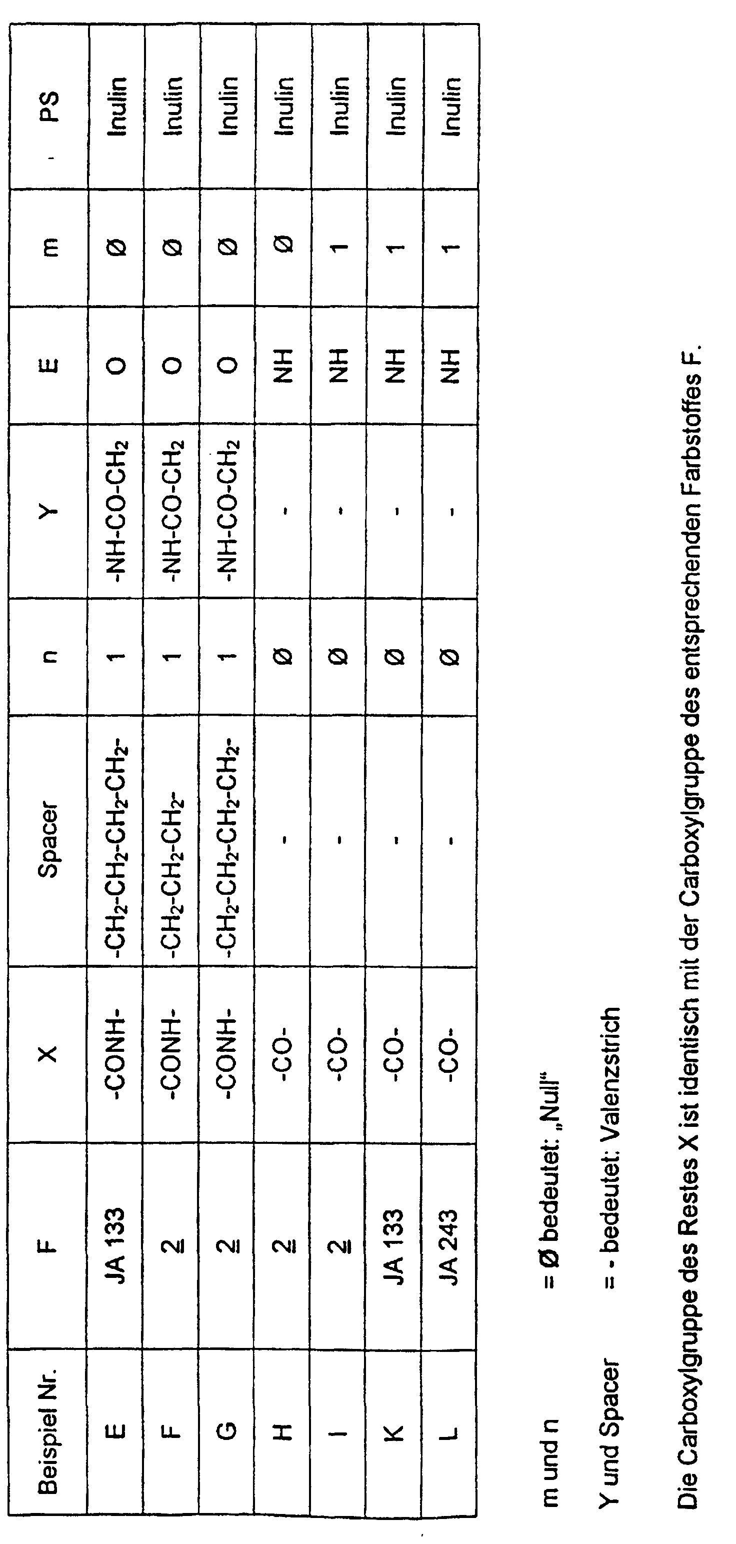

## Patentansprüche

1. Verbindungen der Formel I
F-X-(Spacer)ₙ-Y-E-(CH₂)ₘ-PS (I),
in der
F = eine Farbstoffkomponente mit einem Absorptionsmaximum zwischen 500 und 1300 nm, vorzugsweise zwischen 600 und 900 nm,
n = 0 oder 1,
m = 0 oder l,
X für den Fall, daß n = 0 ist, =
-CO, -NH-C=O, -NH-C=S, -NH-C=NH, -CONH-C=S,
X für den Fall, daß n = 1 ist, =
NH, -CONH, -SO₂NH, -NHCONH, -NHCSNH,
Spacer = eine C₂-C₆ Alkylengruppe oder die Gruppe 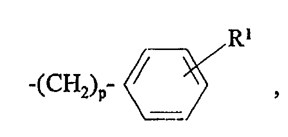 wobei
p = 0 - 4 und R₁ = Wasserstoff oder die Sulfonsäuregruppe sein kann,
Y = Valenzstrich, -CH₂, -NHCOCH₂, -NH-C=O, -NH-C=S, -NH-C=NH,
E = O oder NH
und PS = ein Polyfructosan darstellt, wobei im Falle, daß E = O und m = 0 ist, das Sauerstoffatom , über das das Polysaccharid gebunden ist, Teil dieses Polysaccharids ist, und im Falle, daß E = NH ist, es sich um ein aminiertes bzw. aminoalkyliertes Polyfructosan handelt.

2. Verbindungen der Formel I gemäß Anspruch 1, in denen
X = -CO, -NH, -CONH, -SO₂NH, -NH-C=O, -NH-C=S,
Spacer = eine C₂ - C₆ Alkylengruppe oder die Gruppe 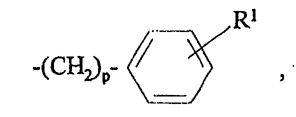 wobei
p bevorzugt 0 oder 1 ist und R₁ Wasserstoff darstellt,
n = 0 oder 1,
Y = Valenzstrich, -CH₂, -NH-COCH₂, -NH-C=O,
m = 0 oder 1,
E = O oder NH
und PS vorzugsweise der Sinistrin-, Inulin-Rest oder ein aminiertes oder aminoalkyliertes Derivat von Sinistrin oder Inulin darstellen.

3. Diagnostisches Mittel, enthaltend eine Verbindung gemäß Anspruch 1 oder 2.

4. Diagnostisches Mittel gemäß Anspruch 3, dadurch gekennnzeichnet, daß es zur Bestimmung der glomerulären Filtrationsrate beim Menschen eingesetzt wird.

5. Verwendung von Verbindungen gemäß Anspruch 1 oder 2 zur Herstellung eines Diagnostikums zur Bestimmung der glomerulären Filtrationsrate beim Menschen.

6. Verwendung eines Farbstoffes gemäß Anspruch 1 oder 2 zur Herstellung eines Diagnostikums zur Bestimmung der glomerulären Filtrationsrate, **dadurch gekennzeichnet, daß** man in zeitlichen Abständen oder kontinuierlich die Konzentration des Farbstoffes bestimmt.

7. Verwendung gemäß Anspruch 6, **dadurch gekennzeichnet, daß** die Bestimmung nicht invasiv erfolgt.

## Claims

1. Compounds of formula I
F-X-(spacer)ₙ-Y-E-(CH₂)ₘ-PS (I),
in which
F is a dye component with an absorption maximum between 500 and 1300 nm, preferably between 600 and 900 nm,
n = 0 or 1,
m = 0 or 1,
if n = 0, X =
-CO, -NH-C=O, -NH-C=S, -NH-C=NH, -CONH-C=S,
if n = 1, X =
NH, -CONH, -SO₂NH, -NHCONH, -NHCSNH,
spacer = a C₂-C₆ alkylene group or the group 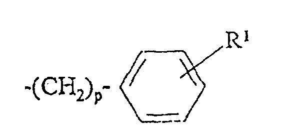 in which p = 0 - 4 and R₁ = hydrogen or can be a sulfonic acid group,
Y = a valence dash, -CH₂, -NHCOCH₂, -NH-C=O,
-NH-C=S, -NH-C=NH,
E = O or NH
and PS = a polyfructosan and if E = O and m = 0, the oxygen atom via which the polysaccharide is bound is part of this polysaccharide, and if E = NH it is an aminated or aminoalkylated polyfructosan.

2. Compounds of formula I as claimed in claim 1, in which
X = -CO, -NH, -CONH, -SO₂NH, -NH-C=O, -NH-C=S,
spacer = a C₂-C₆ alkylene group or a group 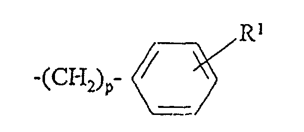 in which
p is preferably 0 or 1 and R₁ represents hydrogen,
n = 0 or 1,
Y = valence dash, -CH₂, -NH-COCH₂, -NH-C=O,
m = 0 or 1
E = O or NH
and PS preferably represents a sinistrin or inulin residue, or an aminated or aminoalkylated derivative of sinistrin or inulin.

3. Diagnostic agent containing a compound as claimed in claim 1 or 2.

4. Diagnostic agent as claimed in claim 3, wherein it is used to determine the glomerular filtration rate in humans.

5. Use of compounds as claimed in claim 1 or 2 for the production of a diagnostic agent to determine the glomerular filtration rate in humans.

6. Use of a dye as claimed in claim 1 or 2 for the production of a diagnostic agent to determine the glomerular filtration rate, wherein the concentration of the dye is determined at intervals or continuously.

7. Use as claimed in claim 6, wherein the determination is not carried out invasively.

## Revendications

1. Composés de formule I
F-X-(Espaceur)ₙ-Y-E-(CH₂)ₘ-PS (I)
dans laquelle
F = un composant de colorant présentant un maximum d'absorption entre 500 et 1300 nm, de préférence entre 600 et 900 nm,
n = 0 ou 1,
m = 0 ou 1,
X représente, pour le cas où n = 0,
-CO, -NH-C=O, -NH-C=S, -NH-C=NH, -CONH-C=S,
X représente, pour le cas où n = 1,
NH, -CONH, -SO₂NH, -NHCONH, -NHCSNH,
espaceur = un groupe alkylène en C₂ à C₆ ou le groupe 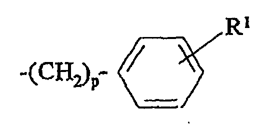 où
p = 0 à 4 et R₁ peut être un atome d'hydrogène ou le groupe acide sulfonique,
Y = un trait de valence, -CH₂, -NHCOCH₂, -NH-C=O, -NH-C=S, -NH-C=NH,
E = O ou NH
et PS = un polyfructosane, où, dans le cas où E = O et m = 0, l'atome d'oxygène, via lequel le polysaccharide est lié, est une partie de ce polysaccharide et, dans le cas où E = NH, il s'agit d'un polyfructosane aminé ou aminoalkylé.

2. Composés de formule I selon la revendication 1, dans lesquels
X = -CO, -NH, -CONH, -SO₂NH, -NH-C=O, -NH-C=S
espaceur = un groupe alkylène en C₂ à C₆ ou le groupe 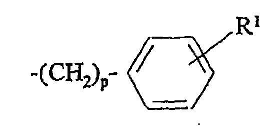 où
p représente de préférence 0 ou 1 et R₁ représente un atome d'hydrogène,
n = 0 ou 1
Y = un trait de valence, -CH₂, -NH-COCH₂, -NH-C=O,
m = 0 ou 1
E = O ou NH
et PS représente de préférence le reste sinistrine, inuline ou un dérivé aminé ou aminoalkylé de la sinistrine ou de l'inuline.

3. Agent diagnostique contenant un composé selon la revendication 1 ou 2.

4. Agent diagnostique selon la revendication 3, **caractérisé en ce qu'**il est utilisé pour la détermination du taux de filtration glomérulaire chez les êtres humains.

5. Utilisation de composés selon la revendication 1 ou 2 pour la préparation d'un agent diagnostique pour la détermination du taux de filtration glomérulaire chez les êtres humains.

6. Utilisation d'un colorant selon la revendication 1 ou 2 pour la préparation d'un agent diagnostique pour la détermination du taux de filtration glomérulaire, **caractérisé en ce qu'**on détermine la concentration en colorant à des intervalles espacés dans le temps ou en continu.

7. Utilisation selon la revendication 6, **caractérisé en ce que** la détermination a lieu de manière non invasive.
